# EUROPEAN PATENT SPECIFICATION

(11) **EP 1 963 482 B1**
(45) Date of publication and mention of the grant of the patent: **23.05.2012**
(21) Application number: 06829777.9
(22) Date of filing: 20.12.2006
(51) Int. Cl.: C12N 1/10, C07K 16/20, A61K 35/68, A61K 39/012

(54) **AVIRULENT ISOLATE OF NEOSPORA CANINUM AND ITS USES**
AVIRULENTES ISOLAT VON NEOSPORA CANINUM UND VERWENDUNGEN DAVON
ISOLAT AVIRULENT DE NEOSPORA CANINUM ET SES UTILISATIONS

(30) Priority: 23.12.2005 ES 200503163
(43) Date of publication of application: 03.09.2008
(73) Proprietor: LABORATORIOS HIPRA, S.A., 17170 Amer (ES)
(72) Inventor: ORTEGA MORA, Luis Miguel, E-28240 Hoyo de Manzanares (ES); COLLANTES FERNANDEZ, Esther, E-28043 Madrid (ES); ROJO MONTEJO, Silvia, E-28923 Alcorcon (ES); ALVAREZ GARCIA, Gema, E-28935 Mostoles (ES); REGIDOR CERRILLO, Javier, E-28025 Madrid (ES)
(74) Representative: Sugrañes Patentes y Marcas
(86) International application number: PCT/EP2006/012296
(87) International publication number: WO 2007/073914

(56) References cited:
- WO-A-02/10340
- DATABASE EM EST 20 November 2003 COLE R. ET AL.: 'NcEST3e25b11.y1 Nc-LIV Tachyzoite cDNA Library Neospora caninum cDNS 5' mRNA sequence' Database accession no. CF937360
- DATABASE EM EST 16 January 2001 COLE R. ET AL.: 'NcEST3a23g02.y1 Nc 1314 Tachyzoite cDNA Neospora caninum cDNA 5', mRNA sequence' Database accession no. BF824453

### SEQUENCE LISTING

<110> Laboratorios HIPRA, S.A.
<120> Avirulent isolate of *Neospora caninum* and its uses
<130>
<160> 12
<170> PatentIn version 3.3
<210> 1
   <211> 299
   <212> DNA
   <213> *Neospora caninum*
<400> 1 
tttcaactgt caacagatac ttttttctag ttcgactcac cgcaacacat gcccacagcg 60

acagatacgc gccatatgca ggcgcggca ggagcatgca cgcttgcgac aagtatatat 120

ataaatatat atatgtatat atgtataaat atatatatat gtatatatgt ataaatatat 180

atatatatat gtatatatgt ataaatatat atatatatgt atatgtatat atatgcaagg 240

ccggtgtgtg gaccttcggc tgaagctgag gagttcgtcg aggtgtatgg cgagtgatg 299
<210> 2
   <211> 228
   <212> DNA
   <213> *Neospora caninum*
<400> 1 
gaaagacgat tccccatttc aaactgcaaa cgtctcagca aatacaagtg acctcttgcg 60

cccggagttc ttcgtatgca cacacatcta catatatata tatatataca tatatgcatt 120

tccatatgga tttacatgtg tagaaaatat agatgtgtat gtttgatttt gtgactctcg 180

atgcatctgc ctgcacgcgc atgtggaggt tttcttgtga atgcagca 228
<210> 3
   <211> 202
   <212> DNA
   <213> *Neospora caninum*
<400> 1 
cggtagaggc gaagtcgaac tcctctgctg ttttattgga cgcgagggtc tcaccgaaaa 60

gctgcatata tatatatttg tatcatatat atatatatat atatatgtat atcccaagta 120

accgcggcat gtgtggactc cacacctagt ctttagacac aacttcctgc cccagagtgt 180

ctgcctgcgt cttccgttct cg 202
<210> 4
   <211> 186
   <212> DNA
   <213> *Neospora caninum*
<400> 1 
ccactgtcgt atgaatgcat atatatatat atatatatat aattatgtag atgtacctgt 60

atctatacat atgtacgtgt gccttgattg ctgcaccatg ccgttttgtg gcctgtttta 120

cgaaggagaa gggattcccc tttcatttca tttgcagaaa attgacaaga gctgcagcct 180

caccat 186
<210> 5
   <211> 235
   <212> DNA
   <213> *Neospora caninum*
<400> 1 
cggcgtagtg catcttctgt ttttatctgt cgaaagacgc ttggcgtcac cacacttttt 60

cctttgcata ttggcacact tgtacgcgca tactgttcgc acatctacac ctctgcatat 120

atatatatat atatatatat atacatttat atacgtgggc gtataagcgt gggcgtgttt 180

gggcagtgcg cgcggtgtac gtgggaggag aggggcgggt ttcgcgacgc ttgac 235
<210> 6
   <211> 267
   <212> DNA
   <213> *Neospora caninum*
<400> 1 
tcaggactcc gtcacaccgg tgaaggcagt ctcgtctgat atggcgcaca catgacagct 60

ttaacacgtg cgtatgtaaa taggcatgcg agagaatacc ccggtttacg tggacagacc 120

tgacgggctg aaagtcccag acggtcgacg gtgacataag gcggatacgc gcagagatgt 180

gcgtatatat atatatatat atgtaggcct ttcgcctggg cgctgccacg ttgacacgct 240

agtactttgt ctcggggcga ggaggcg 267
<210> 7
   <211> 391
   <212> DNA
   <213> *Neospora caninum*
<900> 1 
gagctcgcca atagatctga ggaaccctca aagtgcaaag tgcatgtttg tatcccttga 60

agctatatat atatatatat atatatatat atatatataa cttcatatgc atatgaatgt 120

ctggtctcgc actcatggga ttttgtaggc cgatgcgtgc ttacccatcg acccctctgc 180

atagctgtcc atctgtgtgt ctgcataata tgcacacatg catctaaagc tgcgtgcatg 240

taactattat tcgtgcatgc gcaaaatact gacatccata tatatatata tatatatata 300

tgtacatgca taccacgtgt gtacgattac gttagcagag gcggccgatt gcgcgaccac 360

gacagagggg ttggaccgtg gtcatctttg t 391
<210> 8
   <211> 219
   <212> DNA
   <213> *Neospora caninum*
<400> 1 
cacgacagtg actctccata catcccgcgc tgctcccgtc gctgcacact tgcatgcacc 60

tatacgtctt cagaccataa tatatatata tatatatata tatatagggg ataggcgacg 120

acggcgagtg ttgcgtgtgt gcgttccctg cggagagagc gctagggagt cgattgacag 180

gggctgtggg ggcctgctta cgggagagga gggactgaa 219
<210> 9
   <211> 193
   <212> DNA
   <213> *Neospora caninum*
<400> 1 
agcgcgaaac tcttgccaac agatttccac gtgtctcttc cgtgtacggc tattgcaagg 60

cacaaaggtc cccgaaaaat ttcatgctga catatatata tatatatata tatatatata 120

tatggatacg cttctttcaa agttggcagt tttgactgaa gctttttcg aatgcggaat 180

cccgtctttc cgc 193
<210> 10
   <211> 279
   <212> DNA
   <213> *Neospora caninum*
<900> 1 
ttctctccgc tttctccaaa tttaaaaggc ctttcggaag tctcccgagc tgtgacctcc 60

ctcgtgtcgt actcgtagta gtagtactac tactactact actagaagaa gaagaagaag 120

aagaagaaga agaagaagaa gaagaagaag aagaagaaga agaagatgat gatgatgatg 180
 
atgatgatga tgatgacaat ctacgtcaat ctacgtcaaa cagggatgtg caagtacctg 240

caaggaagag ctataggaag agagaaagga gaataaagg 279
<210> 11
   <211> 241
   <212> DNA
   <213> *Neospora caninum*
<400> 1 
tgtttttttg agttccttgc aaaactcggg agaaattcgg gacccaaggg gaagtggaca 60

ctcgggggc tgagggcaac cgtctcgttt ctggccagga gcaaaagcta ctcaagagac 120

agcaagatcg gcgcatttcc gctccgatgc tctgcgtgtg tgtgtgtgtg tgtgtgtgtg 180

tgtgtgtgcg cccgagtctc gtttgttatt aattgccgtc gtctgcgtgt tcggagtctc 240

ttgtacc 247
<210> 12
   <211> 248
   <212> DNA
   <213> *Neospora caninum*
<400> 1

ggagcccggt actcctggat ggtgagacgg ttgaatcggg ctggtcccta atcgtttacc 60

acgagttccc tccaacatgc atatatacat gtacatacat acatacctac atacatacat 120

acatacatac atttcccccc gctctttctc aaacgtttcc tttggccgag cttgcacata 180

tacaaagtat aacatccagg cggcgatgga atcagcatgt tcacttttgg tgaaggggag 240

ggtagaaa 248

## Description

### Technical field

The present invention relates to a new isolate of *Neospora caninum,* pharmaceutical compositions for the prevention and treatment of neosporosis in animals, and the development of diagnostic tests to detect infection by *Neospora caninum* therein.

### Prior art

*Neospora caninum* is a parasite of the *Sarcocystidae* family belonging to the Phylum Apicomplexa, within which some of the most important diseases causing organisms known by humans are grouped. This group includes genera such as *Plasmodium, Babesia, Cryptosporidium, Eimeria* and *Toxoplasma.*

*N. caninum* has been implicated as a producing agent of miscarriage and neonatal mortality in cattle, although the infection has also been described in other ungulates and canines, such as the coyote and the dog, who act as definitive hosts in this parasite's biological cycle. It undoubtedly highlights the importance of this disease in cattle and dogs.

Bovine neosporosis is considered as a parasitic disease of cosmopolitan distribution and represents one of the most frequent causes of reproductive failure in the various countries where it has been studied, among them Spain.

The most important clinical manifestation of the infection is miscarriage in gestating females which generally takes place between the third and ninth month of gestation. In relation to the transmission of the disease, it highlights the importance of congenital transmission and the relatively little importance of postnatal transmission.

With regard to the biological cycle of *N. caninum,* three stages have been described to date:
- oocysts which are eliminated in the faeces of the definitive host, and which are capable of infecting the host after their ingestion, as a process of germination and release of the infective sporozoites.
- tachyzoites, responsible for the acute phase of the infection which quickly multiply in the host cells, producing cell lysis, tissue lesions and their propagation to other tissues, and
- bradyzoites, which multiply slowly and are found forming tissue cysts in the host cells, and are responsible for the chronic phase of the infection.

The diagnosis methods used to detect infection by *N. caninum,* mainly in adult cattle, are constituted by techniques based on the detection of specific serum antibodies against the parasite.

To do this, different serological tests have been designed which include indirect immunofluorescence (IIF), immunoenzymatic assays (ELISA) and immunoblot.

The antigens used in these serological tests come from tachyzoites obtained in cell cultures of bovine and canine isolates of *N. caninum.* Said tachyzoites are used in various ways: whole, cellular extracts obtained therefrom, and recombinant antigenic proteins or affinity-purified by cellular extracts of tachyzoites.

Patent application PCT WO-A-97/39009 discloses a method to diagnose neosporosis which uses an antigenic protein derived from *N. caninum* tachyzoites.

It should be taken into consideration that although positive serological tests help to identify the infected adult animal, a negative result does not definitively reject injection.

Different studies have demonstrated that serum antibodies against *N*. *caninum* may fluctuate depending on the age and state of gestation in cattle, which enormously hinders interpretation of the results. The need persists, therefore, for alternative diagnostic methods to detect infection by *N. caninum.*

The state of the art discloses various vaccine products for protection against neosporosis which include inactivated vaccines, vaccines developed from recombinant antigenic protein or from DNA, and live vaccines.

Patent application WO-A-99/20303 discloses a vaccine which comprises inactivated tachyzoites and the adjuvant POLYGEN, which generates an immune response in cattle. In later studies it has been seen that said vaccine was incapable of protecting against miscarriage in experimentally infected gestating reproducers. Currently, it is the only vaccine against *N. caninum* registered in the United States of America, and is marketed under the name of NEOGUARD.

Patent application PCT WO-A-95/25541 discloses a vaccine against neosporosis which comprises cell cultures of tachyzoites of a *Neospora caninum* isolate.

Patent application EP-A-0898969 proposes a vaccine which includes a homogenate of *N. caninum* tachyzoites, wherein no viable cells are found.

Patent application EP-A-0953641 discloses a vaccine which contains a *Neospora caninum* protein selected from the group formed by GRA1, GRA2, SAG1, MIC1 or MAG1, or a polynucleotide that codes for said proteins.

As disclosed in Patent application WO-A-2004/026903, the main drawback of inactivated vaccines is that they often do not provide sufficient cellular immunity, require adjuvant agents, do not provide local immunity and are dangerous if the inactivation is not total.

Patent application EP-A-0841392 discloses a vaccine which comprises live cells from temperature-sensitive *N. caninum* mutants which are capable of preventing the development of clinical symptoms associated with neosporosis in a challenge test in an experimental murine model.

As disclosed in Patent application WO-A-2004/026903, the live attenuated vaccines have a number of advantages such as: the activation of all phases of the immune system, the induction of humoral IgG and local IgA, the generation of immune responses to multiple protective antigens, and to provide longer lasting immunity. Among the drawbacks we should highlight the difficulty in establishing the appropriate level of attenuation and the possibility of reversion to virulence.

Hence the importance of identifying and isolating parasites that have a high degree of attenuation in a natural manner.

In the comparative studies of the biological properties of different isolates, the existence of variability has been observed, which includes differences in pathogenicity and genetic profiles, which implies that not all *N. caninum* isolates have the same properties or biological behaviour.

For example Atkinson, in et al., 1999, Parasitology 118 (Pt 4):363-370, and in Miller et al., 2002, Aust Vet J. 80(10):620-625, an experimental murine model was used which revealed the existence of differences in the virulence of the isolates called Nc-SweB1, Nc-1 and Nc-Nowra against the isolate called Nc-Liv, a parasite which produces more severe clinical signs.

Patent application PCT WO-A-02/103430 discloses vaccines which comprise live tachyzoites of a *Neospora* isolate called Nc-Nowra. Said parasite was isolated in Australia, and has a naturally attenuated pathogenicity.

Taking into consideration that there are no effective treatments available for neosporosis, there persists the need for a vaccine which may induce a suitable immune response to prevent and treat neosporosis, and which does not have the drawbacks of the mentioned vaccines.

### Object of the invention

The authors of the invention have isolated and identified a new strain of *Neospora caninum* which is naturally attenuated, and which is suitable for the preparation of pharmaceutical compositions and the development of diagnostic methods.

The object of the present invention is a parasite isolate deposited in the culture Collection of Algae and Protozoa (CCAP) with access number CCAP 2051/2 which has a high degree of natural attenuation.

In a second aspect, the invention also has the object of a biologically pure cell culture infected by the isolate of the invention.

In a third aspect, the invention also has the object of an antibody which reacts specifically against an antigen from the isolate of the invention characterized in that the antigen is selected from the group formed by the isolate, tachyzoites, bradyzoites and sporozoites.

In a fourth aspect, the invention also has the object of a vaccine which comprises the isolate of the invention.

In a fifth aspect, the invention also has the object of the use of the isolate of the invention to prepare a vaccine for the treatment and/or prevention of neosporosis in animals.

In an sixth aspect, the invention also has the object of the use of antibodies which specifically react against an antigen from the parasite isolate of the invention characterized in that the antigen is selected from the group formed by the isolate, tachyzoites, bradyzoites and sporozoites to prepare a drug for the treatment of an infection and/or disease caused by *Neospora caninum* in animals.

In a seventh aspect, the invention also has the object of the use of antigens from the isolate of the invention characterized in that the antigen is selected from the group formed by the isolate, tachyzoites, bradyzoites and sporozoites to detect infection by the parasite *Neospora caninum* in animals, from a biological sample.

In a eighth aspect, the invention also has the object of the use of antibodies which specifically react against an antigen from the parasite isolate of the invention characterized in that the antigen is selected from the group formed by the isolate, tachyzoites, bradyzoites and sporozoites to detect the infection by the *Neospora caninum* parasite in animals, from a biological sample.

### Description of the figures

Figure 1

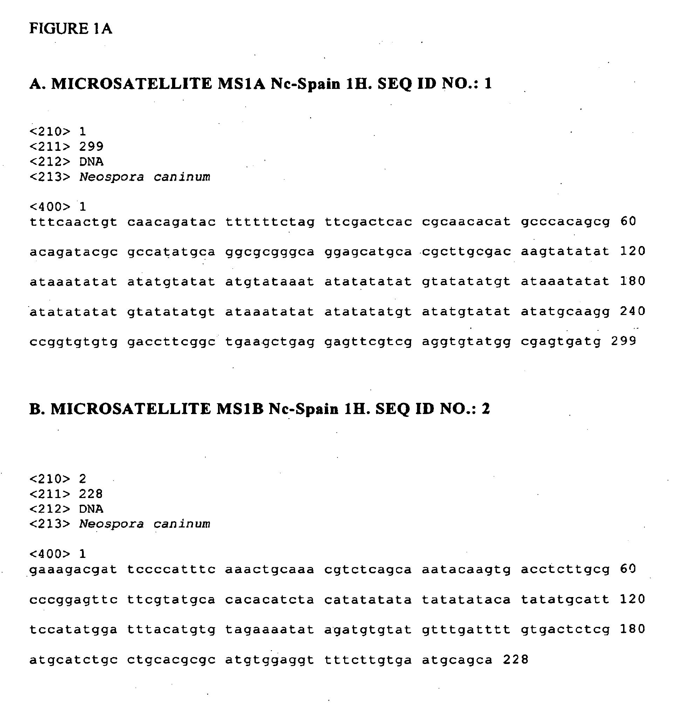

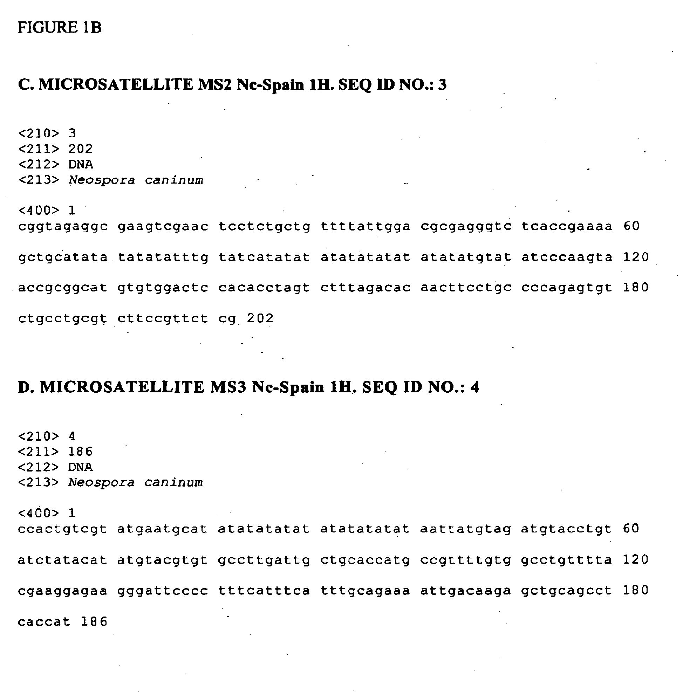

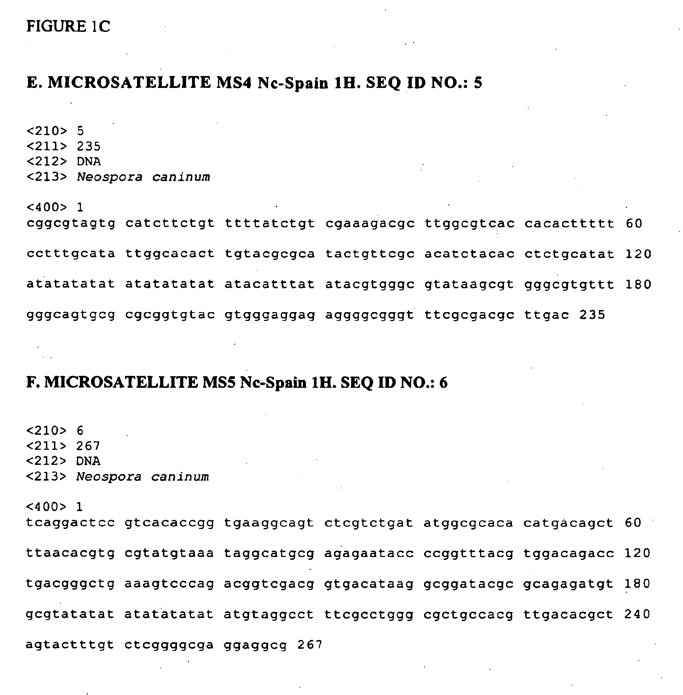

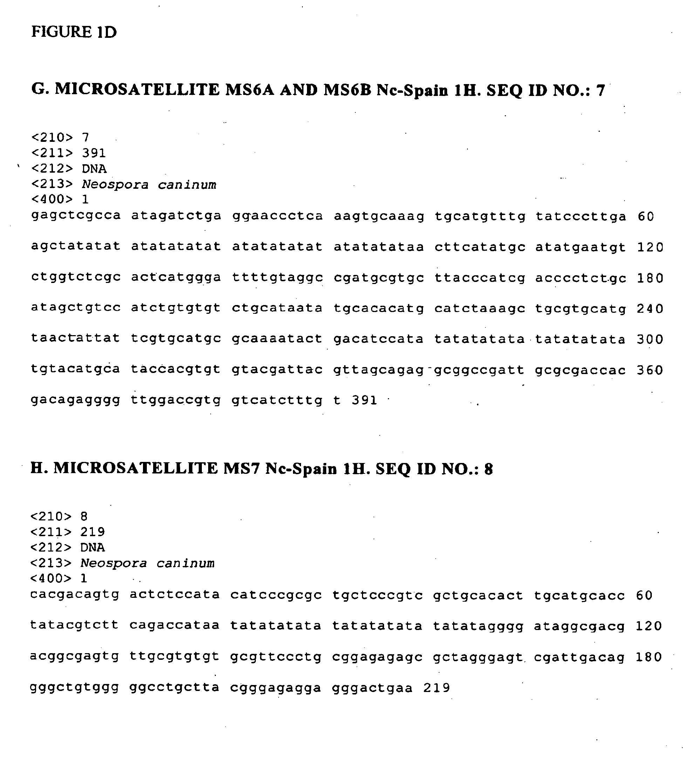

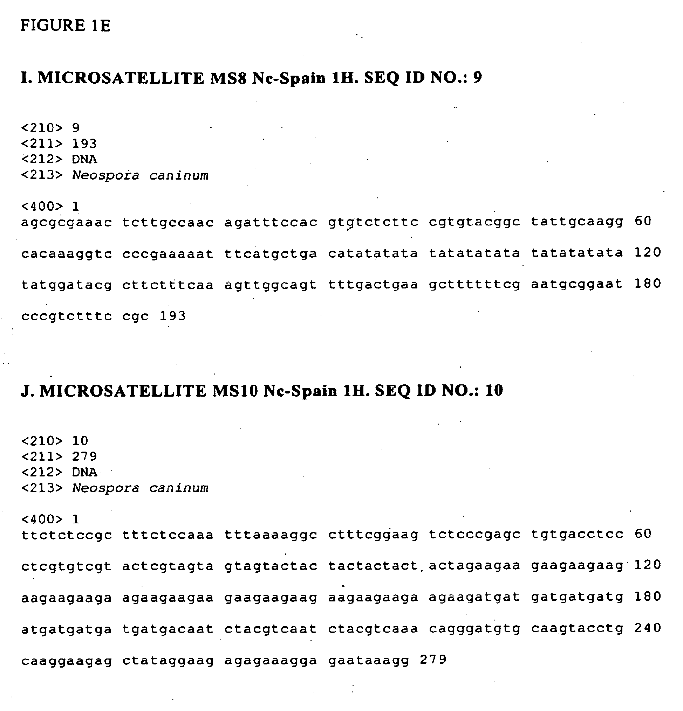

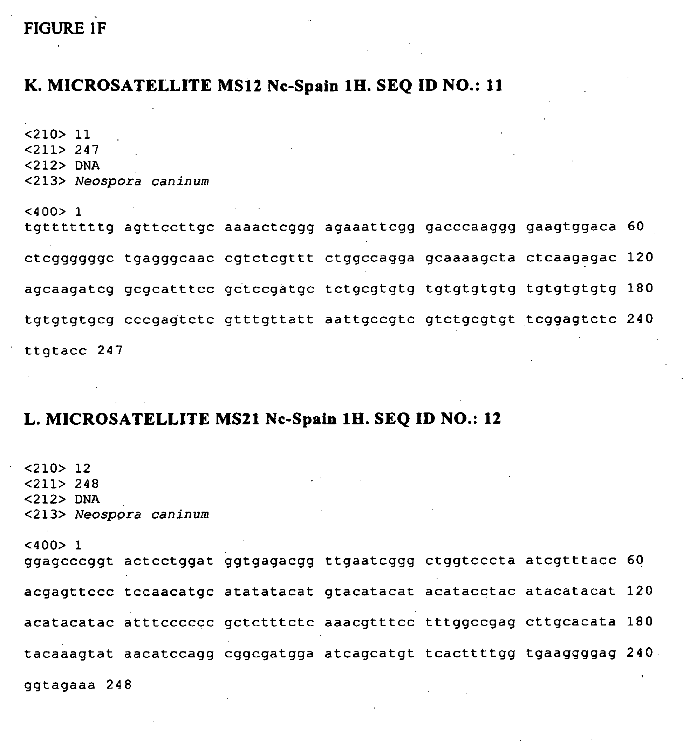

   Figures 1A a 1F show the nucleotide sequences of DNA of each one of the 13 amplified and sequenced microsatellites of the isolate object of the invention Nc-Spain 1H. Figure 1A: A. MS1A (SEQ. ID. NO.:1), B. MS1 B (SEQ. ID. NO.:2); Figure 1B: C. MS2 (SEQ. ID. NO.:3), D. MS3 (SEQ. ID. NO.:4); Figure 1C: E. MS4 (SEQ. ID. NO.:5), F. MS5 (SEQ. ID. NO.:6); Figure 1D: G. MS6A and MS6B (SEQ. ID. NO.:7), H. MS7 (SEQ. ID. NO.:8); Figure 1 E: I. MS8 (SEQ. ID. NO.:9), J. MS10 (SEQ. ID. NO.:10); and Figure 1 F: K. MS12 (SEQ. ID. NO.:11) and M. MS21 (SEQ. ID. NO.:12).
Figure 2

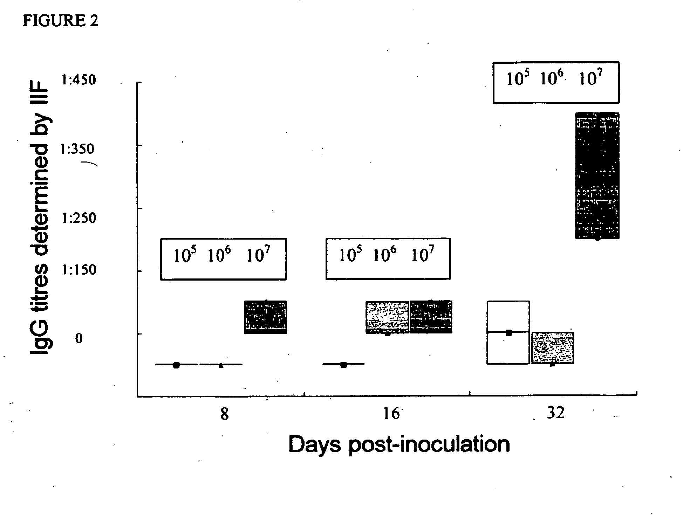

   Figure 2 shows the evaluation of the humoral immune response in the murine experimental infection model of the Nc-Spain 1H isolate. The y-axis represents the titres of IgG determined using the IIF technique on days 8, 16 and 32 post-inoculation in mice which were inoculated with 10⁵, 1 O⁶ and 10⁷ tachyzoites of the Nc-Spain 1H isolate. The bars represent the median, and the quartiles 25% and 75% for each case.
Figure 3

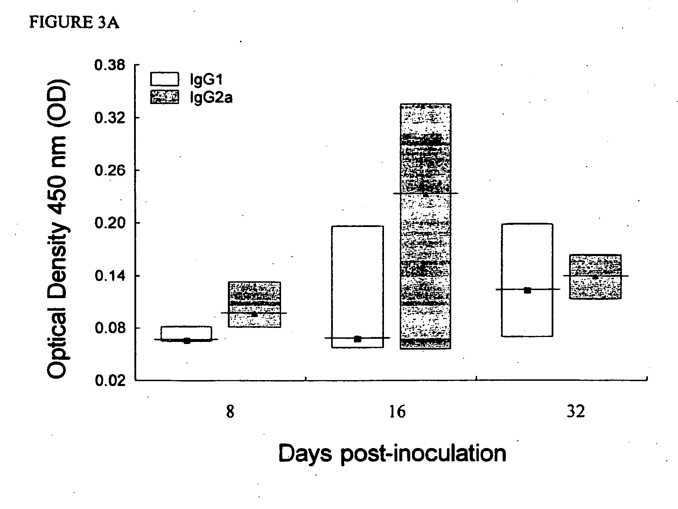

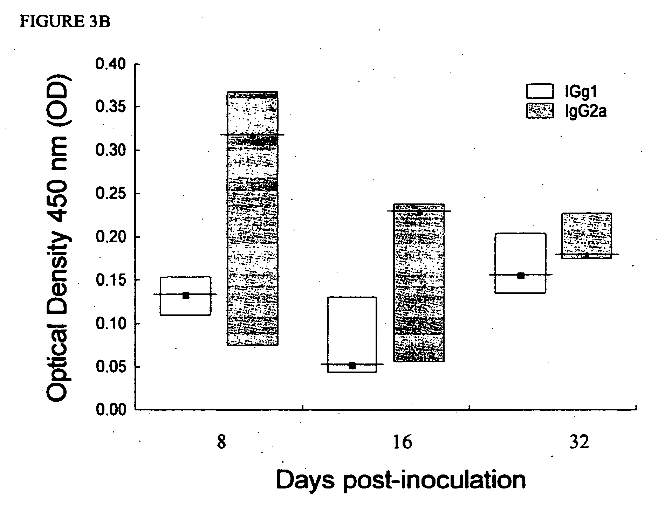

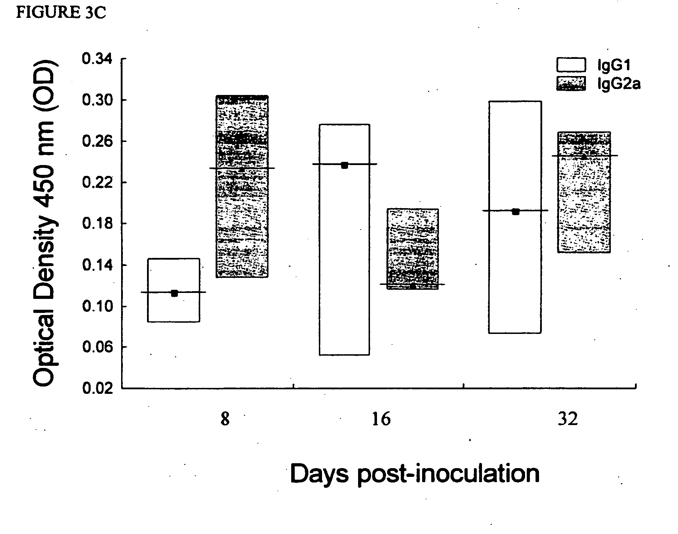

   Figures 3A, 3B and 3C show the evaluation of the humoral immune response in the murine experimental infection model of the Nc-Spain 1H isolate. The y-axis represents the optical density determined at 450 nm, and which is a measurement of the specific IgG1 and IgG2a antibodies against *N. caninum* determined by ELISA on days 8, 16 and 32 post-inoculation produced in the mice of Groups A (Figure 3A), B (Figure 3B), and C (Figure 3C) which were inoculated with 10⁵, 10⁶ and 10⁷ tachyzoites of the Nc-Spain 1H isolate, respectively. The bars represent the median, and the quartiles 25% and 75%.
Figure 4

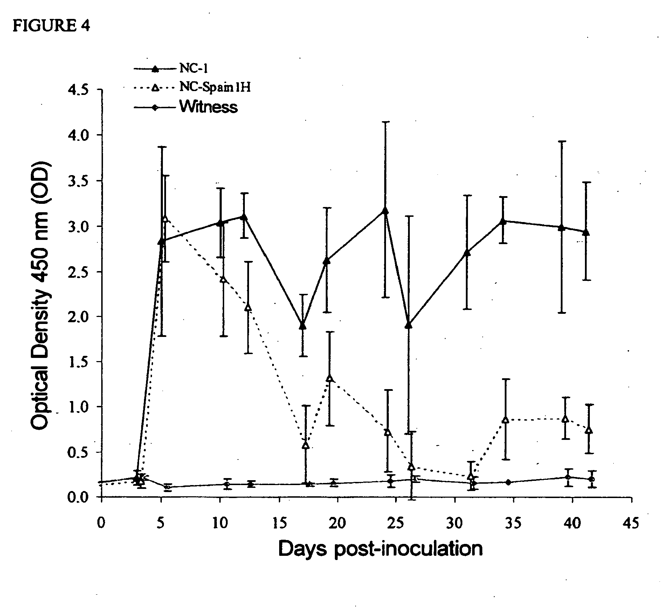

   Figure 4 shows the evaluation of the cellular immune response in the bovine experimental infection model of the Nc-Spain 1H isolate. The y-axis represents the optical density determined at 450 nm, which is a measurement of the IFN-γ antibodies produced by animals inoculated with 10⁷ tachyzoites of the reference isolate Nc-1, of the Nc-Spain 1H isolate and PBS (control group). The x-axis shows the post-inoculation days.
Figure 5

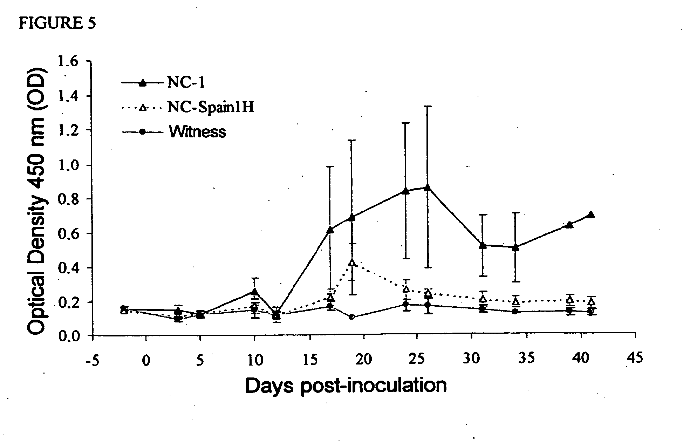

   Figure 5 shows the evaluation of the humoral immune response in the bovine experimental infection model of the Nc-Spain 1H isolate. The y-axis represents the optical density determined at 450 nm (OD), which is a measurement of the mean values of the IgG anti-*N*. *caninum* antibodies produced by cattle inoculated with 10⁷ tachyzoites of the reference isolate Nc-1, of the Nc-Spain 1H isolate and PBS (control group). The x-axis shows the post-inoculation days.
Figure 6

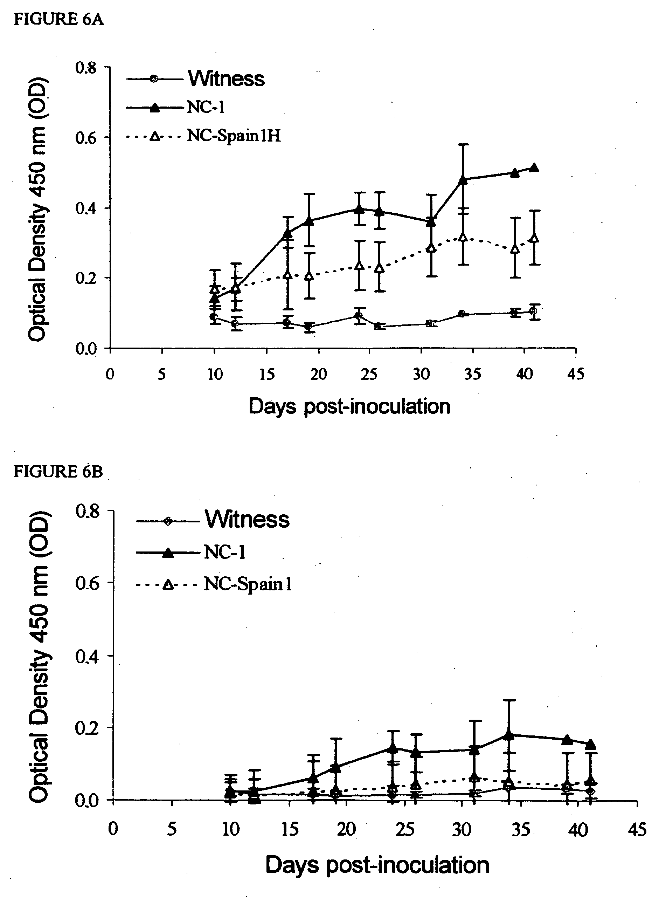

   Figures 6A and 6B show the evaluation of the humoral immune response in the bovine experimental infection model of the Nc-Spain 1H isolate. The y-axis represents the optical density determined at 450 nm (OD), which corresponds to the mean values of the IgG1 (Figure 6A) and IgG2 (Figure 6B) anti-*N*. *caninum* antibodies produced by the animals inoculated with 10⁷ tachyzoites of the reference isolate Nc-1, of the Nc-Spain 1H isolate and PBS (control group). The y-axis shows the post-inoculation days.

### Detailed description of the invention

In this description when we speak of the parasite isolate or simply isolate of the invention it relates to the isolated strain of *Neospora caninum* at any stage of its biological cycle which includes tachyzoites, bradyzoites and sporozoites.

### New parasite isolate

The object of the present invention is a new strain of *Neospora caninum* isolated from the nerve tissue of a calf of the Frisian-Holstein breed which is congenitally infected.

The new isolate has been called Nc-Spain 1H.

Said isolate has been identified and characterized by different *in vitro* studies and *in vivo* experimental infections as a new isolate of *Neospora caninum.*

The isolate of the invention has a high degree of natural attenuation, in other worlds, it shows attenuation without having been manipulated, and is therefore an appropriate candidate for the development of vaccines against neosporosis, and for the development of diagnostic tests to detect the presence of the parasite or infection caused thereby from biological samples of animals.

A sample of the Nc-Spain 1H isolate was deposited on the date 20.09.2005, in accordance with the provisions of the Budapest Treaty on the International Recognition of the Deposit of Microorganisms for the Purposes of Patent Procedure, in the Culture Collection of Algae and Protozoa (CCAP) located at the Dunstaffnage Marine Laboratory, Dunbeg, OBAN, Argyll PA37 1QA, Scotland, Great Britain, which has been given access number CCAP 2051/2.

This isolate was obtained from homogenates of the nerve tissue (brain and spinal cord) from a congenitally infected dairy calf, as described in Example1.

For its identification and characterization, genomic DNA was extracted from a sample of nerve tissue, which was subjected to a nested PCR developed for the specific detection of the parasite DNA in clinical samples. The specific amplification of the ITS-1 region, as described in Buxton et al., 1998, J.Comp Pathol. 118: 267-279, confirmed the presence of *Neospora* parasites in said sample.

The genetic characterization of the isolate of the invention was performed using microsatellite technology, which was a suitable tool to apply in the identification and discrimination of the different isolates of *N. caninum,* as described in Example 2 of this description.

The microsatellites are constituted by simple nucleotide sequences of 2 to 5 nucleotides which are repeated in tandem and which are characterized by high polymorphism, mainly in accordance with the number of repetitions of the nucleotide motive they show. This type of sequences has been described in a multitude of prokaryotic and eukaryotic organisms, including protozoa, and they have been extensively used for the genotyping of different apicomplexa parasites such as *Toxoplasma gondii* (Ajzenberg et al., 2002, Int.J.Parasitol. 32:27-38), *Cryptosporidium parvum* and *Cryptosporidium hominis* (Mallon et al., 2003, Infect.Genet.Evol. 3:207-218), *Plasmodium falciparum* (Anderson et al., 2000, Mol.Biol.Evol. 17:1467-1482) and *Theileria parva* (Oura et al., 2003, Int.J.Parasitol. 33:1641-1653).

The results obtained on comparing ten *Neospora* isolates, among them the Nc-Spain 1H isolate of the invention, enable us to conclude that they are all different.

The *in vivo* verification that the parasite was of the *Neospora* genus was performed by the inoculation of athymic mice of the BALB/c strain with different quantities of infected tissue, which led to the appearance of clinical signs compatible with neosporosis. Finally, the parasite was isolated in cell cultures of the MARC-145 cell line (monkey kidney cells).

### Pathoaenic and immunogenic characteristics of the new parasite isolate

The pathogenicity of the isolate of the invention was tested in a gestating murine and bovine model as described below in Examples 3 and 4, respectively.

The mice were divided in four groups, three of which were inoculated with 10⁵, 10⁶ and 10⁷ tachyzoites of the Nc-Spain 1H parasite obtained from cell culture, and the fourth group (control) inoculated with PBS buffer solution.

The mice of the infected groups did not develop signs compatible with infection by *Neospora,* nor mortality during the 32 days' duration of the experiment. This demonstrates the high degree of attenuation of the Nc-Spain 1H strain.

Although in said mice, the presence of *N. caninum* DNA was detected in the blood, lungs and brain, its persistence in these organs was very inferior to that which is observed when virulent strains of *N. caninum* were inoculated which also corroborates the high degree of attenuation of this strain.

In the gestating bovine model, heifers were injected on day 70 of gestation intravenously administering 10⁷ tachyzoites of the reference isolate of *N. caninum,* Nc-1 (Dubey et al., 1988, J. Am. Vet. Med. Assoc. 193:1259-63) (group 1), Nc-Spain 1H (group 2) or PBS (group 3). In the group infected with Nc-1, foetal death occurred in 3 of the 5 heifers inoculated. In the animals belonging to the group infected with Nc-Spain 1H, foetal death was not observed during the 45 days' duration of the experiment. Likewise, in group 2, the parasite DNA was not detected in foetal tissues and the lesions observed in the placenta and foetal organs were less severe than the animals infected with Nc-1.

The immunogenicity assays were performed on a murine model with mice (Example 5), and on a bovine model with cows (Example 6).

In these assays it could be observed that in the animals infected with tachyzoites of the isolate of the invention Nc-Spain 1H, the production of antibodies which reacted with antigens of the reference isolate of *N. caninum,* Nc-1, was induced. This demonstrates the capacity of the Nc-Spain 1H strain of inducing specific immune response to *N. caninum.*

The detection of specific antibodies against *N. caninum* in the plasma of mice infected with tachyzoites from the isolate of the invention was determined using IIF and ELISA techniques.

When the IIF technique was used, inactivated tachyzoites of the reference isolate Nc-1 were employed as antigens.

The presence of antibodies was detected from day 8 post-inoculation (PI) in the groups inoculated with the highest dose (10⁷ tachyzoites), and the IgG titres obtained by IIF also varied in accordance with the infective dose, obtaining the highest ones (1:200 - 1:400) in the mice of the group inoculated with the highest dose, as observed in Figure 2.

The detection of immunoglobulins of isotypes IgG1 and IgG2a specific for *N*. *caninum* in the plasma was performed using an indirect immunoenzymatic assay (ELISA) using, as an antigen, protein from the soluble extract of tachyzoites of the reference isolate Nc-1 from *N. caninum* obtained by sonication.

An increase in the IgG2a and IgG1 isotypes were observed from day 8 PI, the increase in immunoglobulin IgG2a being more evident in the groups infected with 10⁵ and 10⁶ tachyzoites of the isolate of the invention.

On days 16 and 32 PI, the mice inoculated with 10⁵ and 10⁶ tachyzoites had a higher concentration of IgG2a than IgG1. Nevertheless, in the group inoculated with 10⁷ tachyzoites, the production of IgG1 was predominant on day 16.

Figures 3A, 3B and 3C show the results of the optical density readings at 450 nm, which is a measurement of the IgG1 and IgG2 antibodies, for the mice infected with 10⁵, 10⁶ and 10⁷ tachyzoites of the Nc-Spain 1H isolate, respectively.

In light of the results, it can be concluded that infection of mice with tachyzoites of the isolate of the invention induces the production of antibodies which specifically react with antigens of a reference isolate of *N. caninum* such as the Nc-1 isolate.

In the tests with heifers, the production of gamma interferon (IFN-γ) was determined as indicator of the cellular immune response generated in the animals due to infection with tachyzoites of the parasite isolate Nc-Spain 1H, and the humoral immune response was evaluated by the ELISA determination of the immunoglobulins IgG, IgG1 and IgG2.

An increase was detected in IFN-γ levels in the infected groups compared to those of the control group on days 5 and 12 post-inoculation, as can be observed in Figure 4.

With regard to the total specific immunoglobulin IgG, the values of the group of animals infected with tachyzoites from the parasite isolate of the invention were greater than those of the control group, but the differences were not significant. The results are shown in Figure 5.

The IgG1 and IgG2 isotypes were evaluated from the day on which a significant increase in the values of IgG were observed with respect to the control group.

From day 12 post-inoculation, a significant increase in IgG1 occurred, and the highest levels were reached from day 34, these values being significantly different to the values of the control group.

The IgG2 values remained slightly above those of the control group, but no significant differences were found between both. The results corresponding to isotype IgG1 are shown in Figure 6A, and those of isotype IgG2 are shown in Figure 6B.

In light of the results, it can be concluded that the infection of heifers with tachyzoites from the isolate of the invention induces the production of antibodies which specifically react with antigens from a reference isolate of *N. caninum* such as the Nc-1 isolate.

Consequently, the Nc-Spain 1H isolate is a *N. caninum* strain with a high degree of attenuation which induces the production of antibodies which specifically react against *N. caninum* antigens.

### Cell cultures

A biologically pure cell culture infected by the isolate of the invention is also an object of the invention.

The expression "biologically pure cell culture" of the *N. caninum* isolate refers to a culture of the Nc-Spain 1H isolate continuously maintained substantially free from other organisms except the host cells where the *Neospora* parasite grows. It is considered that a culture is substantially free from other organisms if, after the procedures of normalized recovery of the isolate, they give the result of a preparation with at least 95% and, preferably 99% or more of the organism.

Preferably the infected cell culture is from monkey kidney cells. Among them, we can mention the cell lines MA-104, Vero, and the cell clones C8 and MARC-145.

### Antibodies

An antibody specifically against an antigen from the isolate of the invention also forms part of the object of the invention.

The term "antibody" refers to an immunoglobulin molecule produced by an animal organism capable of bonding to a specific epitope in an antigen. The antibodies may be constituted by a polyclonal mixture or be monoclonal antibodies. They may also be constituted by intact immunoglobulins of natural origin or recombinant nature or fragments of these antibodies which retain their capacity for recognition and bonding of the target antigen and which include Fv, F(ab), and F(ab)₂, as well as simple chains. It can also use antibodies formed by simple chains wherein the heavy chain or the light chain may be combined with other molecules.

The antigen from the Nc-Spain 1H isolate is selected from the group formed by the isolate, tachyzoites, bradyzoites, and sporozoites.

Preferably, the antigen is a tachyzoite.

The antibodies can be monoclonal antibodies or be constituted from a polyclonal mixture.

The monoclonal antibodies are identical antibodies as they are produced by a single type of immune system cell.

Preferably, the antibody of the invention is monoclonal.

The monoclonal antibodies can be obtained by different techniques well described in the state of the art, such as, for example, in Harlow and Lane, 1988, "Antibodies: A Laboratory Manual", Cold Spring Harbor Laboratory Press, Cold Spring Harbor.

The antibodies of the invention are suitable for detecting the presence of the *Neospora caninum* parasite or the infection caused thereby in animals, from biological samples.

### Use of the new isolate in immunogenic compositions

The invention also has the object of a vaccine which comprises the isolate of the invention deposited with access number CCAP 2051/2.

Said vaccine is a pharmaceutical composition which can be used for the treatment and prevention of infections and diseases caused by *N. caninum.*

In the vaccines, the isolate of the invention is preferably in the form of live attenuated isolate, inactivated or fixed, at any stage of its biological cycle.

The *Neospora caninum* strain of the invention can be directly used to prepare the vaccines against neosporosis, since it has a high degree of attenuation. Nevertheless, it can also be additionally attenuated using attenuation techniques which are well known by persons skilled in the art.

The vaccines that contain the isolate of the invention as immunogenic agent are typically prepared as injectable in liquid solutions or suspensions, or in solid form, for their later reconstitution in solution or suspension previous to their administration.

The vaccine can also be formulated as an emulsion or can encapsulate the immunogenic agent in liposomes, or any suitable system. In its formulation, the immunogenic agent can be combined with habitual excipients of pharmaceutical use compatible therewith, such as, for example: water, saline solution, dextrose, glycerol, ethanol or similar, and their combinations.

The vaccine can also contain wetting or emulsifying agents, pH regulators, preservatives and/or adjuvants which increase the effectiveness of the vaccine such as, for example: aluminium compounds (such as, for example, aluminium hydroxide, aluminium phosphate, and aluminium potassium sulphate), beryllium sulphate, silice, caolin, carbon, water/oil or oil/water emulsions, bacterial endotoxin, *Corynebacterium parvum (Propionobacterium acnes), Bordetella pertussis,* polyribonucleotides, sodium alginate, lanolin, vitamin A, saponins, liposomes, DEAE-dextran, copolymers and other synthetic adjuvants.

Cytokines and/or immunomodulators such as IFN-γ can also be incorporated in the vaccine.

The concentration of the immunogenic agent and of the adjuvant compound may vary within a wide margin provided that the quantity of both is effective. Once the vaccine has been prepared, it can be packed in sterile containers and be stored in a suitable way for its stable maintenance during long periods of time, for example, at low temperature or by lyophilization process.

The vaccines that are presented as injectable can be administered parenterally, by subcutaneous, intradermal, endovenous or intramuscular injection.

Other formulations can also be used to administer the vaccine such as, for example, suppositories or oral formulations, for example, capsules or tablets. In these cases, the formulation techniques, the adjuvants and the suitable auxiliary substances are well known by persons skilled in the art.

The vaccines of the present invention can be administered preventively to animals sensitive to infection or the disease in order to induce a more efficient immune response to the parasite.

The vaccine can also be administered for the treatment of the infection or the disease once the first symptoms have appeared.

Various vaccination regimes can also be effective for the immunization of cattle and other animals. Thus, for example, the cattle can be vaccinated during the reproductive period to prevent miscarriage and reduce the possibility of congenital effects.

The use of the isolate of the invention to prepare a vaccine for the treatment and/or prevention of neosporosis in animals also forms part of the invention.

The use of antibodies which react specifically against an antigen from the strain of the invention to prepare a drug for the treatment of an infection and/or disease caused by *N. caninum* in animals also forms part of the object of the invention.

### Use of the new isolate in diagnostic techniques

In this description "biological sample" refers to any sample obtained from live or dead organisms. Examples of biological samples are biological fluids (blood, serum, plasma, urine, semen, ascitic fluid, cerebrospinal fluid and foetal fluids), tissue samples (from brain, spinal fluids, lung, heart, liver and placenta) and cell cultures.

The use of antigens from the isolate of the invention to detect infection by the *Neospora caninum* parasite in animals, from a biological sample characterized in that the antigen is selected from the group formed by the isolate, tachyzoites, bradyzoites and sporozoites also forms part of the object of the invention.

The antigen of the parasite isolate Nc-Spain 1H is selected from the group formed by the isolate, tachyzoites, bradyzoites, and sporozoites.

Preferably, the antigen used is a tachyzoite.

To detect infection by the *Neospora* parasite in animals from a biological sample, processes can be used to detect the immune response generated by infection by *Neospora,* well described in immunology texts, such as, for example, in Brostoff, Male, and Roitt, 2000, Inmunologia, 5th Edition, Editorial Harcourt Brace.

Thus, for example, the antigen of the invention can be immobilized in a solid surface, and the antigen-antibody complex can be detected using anti-immunoglobulin antigens of the species from which the sample being studied comes, marked with fluorescent molecules or those with enzymatic activity.

The use of antibodies which react specifically against an antigen from the parasite isolate of the invention to detect the presence of the *Neospora* parasite in a biological sample also forms part of the object of the invention.

As has also been described, the antigen from the parasite isolate Nc-Spain 1H is selected from the group formed by the isolate, tachyzoites, bradyzoites, and sporozoites.

Preferably, the antigen used is a tachyzoite.

The antibodies can be monoclonal antibodies or be constituted by a polyclonal mixture.

Preferably, the antibody of the invention is monoclonal.

The immunoassays used to measure the anti-*Neospora* antigens or antigens of the parasite can use competitive or non-competitive antigen-antibody bonding assays.

In the competitive assays, the sample to analyse (for example, anti-N. *caninum* antibodies) competes with another marked antibody (for example, an anti-*N. caninum* monoclonal antibody) due to its specific bonding to an antigen (for example, isolated protein of *N. caninum*) bound to a solid surface. The concentration of the marked antibody bound to the antigen will be inversely proportionate to the quantity of free antibody present in the sample.

Non-competitive assays are the most typical, and therein the components of the sample to be analysed which indicate the presence of the parasite are specifically bound to two reagents which form part of the assay. One of them is used to capture the sample's components and remain bound to a solid surface. The second is marked and is used to measure or detect the presence of previously formed complexes.

In another embodiment, the immunoassay can be performed in liquid phase and by a variety of methods which enable the complex formed from the components of the non-bound sample to be separated. These methods include, for example, immunoprecipitation, column chromatography, absorption and addition of magnetic particles covered with a reagent capable of bonding to the complex.

The immunoblot technique can also be to detect the presence of specific antibodies against *Neospora* in a biological sample. This technique is typically applied to detect the presence of specific antibodies of the sample against a particular protein. Said technique involves the separation of the proteins by electrophoresis in acrylamide gels according to their size and/or isoelectric point in a pH gradient, the transfer of the separated proteins to a suitable solid surface (nitrocellulose or nylon membrane) and the incubation of the transferred proteins with the sample that contains the antibodies. This permits the specific bonding of the antibodies present in the sample to the respective proteins present, forming antigen-antibody complexes which are detected by the use of marked anti-immunoglobulin antibodies from the source animals of the sample.

The marking of the reagents used in the previously described tests can be carried out by radioactive marking with the H³, I¹²⁵, C¹⁴ or P³² isotopes or by the bonding of fluorophore ligands, such as, for example, fluorescein and rhodamine, chemiluminescence, such as, for example, luciferase, enzymes, such as, for example, phosphatase, esterase, peroxidase, to the biotin-streptavidin complex and other antibodies that may act as bonding target for a marked compound. Even so, in some assays, the use of marked compounds is not necessary. For example, in binding texts developed to detect the presence of antibodies, the antigen coats particles which bind together in the presence of specific antibodies against that antigen, detecting the formation of the complexes simply by visual inspection or microscopic viewing.

The following examples are given in order to provide persons skilled in the art with a sufficiently clear and complete explanation of the present invention, but they should not be considered as limitations to the essential aspects of the object thereof, as has been stated in the previous sections of the description.

### Examples

### Example 1.- Obtainment of a new isolate of N. caninum from a congenitally infected calf

This example describes the isolation of the *N. caninum* Nc-Spain 1H parasite from a congenitally infected calf from the Frisian-Holstein race.

The calf was born from a cow that had previously given positive results in the detection tests for specific antibodies to *Neospora* kept in a dairy farm located in the province of Madrid, Spain. Since 2001, the farm has been subjected to different studies to confirm the presence and prevalence of infection by *Neospora,* establishing an endemic infection pattern. The presence of DNA from the parasite in the brain using nested PCR from the ITS-1 region was detected in the miscarried foetuses from the farm.

In March 2003, two twin calves were born from one of the cows identified as seropositive, whose precolostral serum was extracted and analysed to detect specific antibodies against *Neospora* using the IIF technique (Pare et al., 1995, J Vet Diagn Invest. 7(2):273-5), giving a titre of 1:500 as a result. Two weeks after their birth, both calves remained healthy and showed no clinical signs. At this date, one of the calves of female sex was sacrificed by an injection of 20-40 ml of T-61 (Embutramide and Mebezonium iodide, Intervet) endovenously, and immediately after, the brain and the upper portion of the spinal cord were extracted in asceptic conditions and kept at 4°C.

Different portions of 5 to 15 grams in weight were collected from both lobes and from the anterior, middle and posterior region of the brain, as well as from the spinal cord, and they were kept at 4º C in PBS buffer solution with antibiotics (2000 Ul/ml Penicillin G, 200 µg/ml Streptomycin) until its subsequent inoculation in athymic mice of the Balb/c *nu*/*nu* strain.

In the brain samples the presence of *N. caninum was* detected therein using PCR, before isolating the parasite.

For this purpose, they removed different portions of approximately 20 mg from all the sections collected and the genomic DNA was extracted, in order to perform the detection of *N. caninum* DNA using nested PCR which amplifies the ITS-1 region of the parasite (Buxton et al., 1998, J.Comp Pathol. 118: 267-279).

A commercial kit was used to extract the DNA: "Genomic-Prep cell and tissue DNA isolation kit" (Amershan Biosciences) following the manufacturer's instructions and 5 µl of the resulting DNA solution (100-200 ng) was used as mould in the nested PCR of the ITS-1 region described below.

The amplification of the ITS-1 region of ribosomal *N. caninum* DNA was performed on a first PCR reaction using the oligos NN1 (5'-TCAACCTTTGAATCCCAA) and NN2 (5'- CGAGCCAAGACATCCATT), and in a second reaction using the oligos NP1 (5'- TACTACTCCCTGTGAGTTG) and NP2 (5'- TCTCTTCCCTCAACGCT). The PCR mixture of (25µl) is composed of 0.15 µM of each oligo (Sigma), 200µM dNTPs (Sigma), 1 x buffer (75mM Tris pH 9, 20mM (NH₄)₂SO₄, 50mM KCl,) (Biotools, Spain), 2mM MgCl₂ (Biotools) and 0.625 units of DNA polymerase (Biotools). In the first amplification reaction add 5 µl of DNA and in the second, 2 µl of the product of the first. Both reactions are carried out in a thermocycler according to the following program: 94° C for 5 min and 25 cycles of 94° C/1 min, 48º C/1 min and 72° C/1,5 min and a last cycle of 72° C for 5 min. The products resulting from the second amplification with a size of 210 pb, are displayed in 1.6% agarose gel in 0.5 TBE (Tris-borate-EDTA buffer) x ethidium bromide stain.

The presence of the parasite was detected in the majority of brain portions analysed.

Once the presence of the parasite was confirmed in the brain samples, they were homogenized with cycles of 1 min. duration in an automatic IUL Masticator until its perfect disintegration; the macerates were filtered through a previously sterilized 150 µm mesh and centrifuged for 10 minutes at 1350 g. The supernatant was discarded and the precipitate equivalent to approximately 5 grams of starting tissue was inoculated intraperitoneally with an insulin syringe and needle with a diameter of 0.5x16 mm in female mice of the Balb/c *nu*/*nu* strain with an approximate weight of 20-25 grams. The entire process was performed in aseptic conditions.

The mice inoculated were observed daily to detect the appearance of clinical signs attributable to neosporosis which include weight loss, nervous disorders, paralysis of the extremities and lethargy, as described in Yamane et al., 1998, J. Vet. Diagn. Invest. 10: 364-368. These clinical signs were observed in all the mice inoculated between 32-40 days post-inoculation (PI). After the appearance of these symptoms, the mice were sacrificed in a CO₂ atmosphere and used for the isolation of the parasite in cell culture, by the inoculation of the peritoneal wash.

Subsequently, the brain of the euthanized mice was removed and it was homogenized in PBS solution with antibiotics-antimycotics (Gibco, BRL, UK) by successive rounds of the syringe with 21 G and 25 G needles.

The homogenate equivalent to half a brain was inoculated in a new mouse of the Balbc *nu*/*nu* strain which was also monitored until the appearance of the clinical signs described. The process was performed up to 7 consecutive rounds of mouse to mouse, observing the appearance of the clinical signs already described in all the mice inoculated between days 28 and 35 PI. In order to confirm that the clinical signs observed were a consequence of infection by *N. caninum,* the DNA was extracted from 20 mg of brain sample of the mice that showed the symptoms and was subjected to the detection of the product of specific amplification of the nested PCR of the ITS-1 region of DNA from the parasite as we have previously described.

With regard to the isolation in cell culture, it was performed by the inoculation of the peritoneal wash of the sacrificed mice on a culture of MARC-145 cells in monolayer.

For this purpose, the peritoneal cavity was washed with approximately 8 ml of Dulbeco's Modified Essential Medium (DMEM) supplemented with 2% bovine foetal serum and a 2% antibiotic-antimycotic solution (100 UI/ml Penicillin G, 100 µg/ml Streptomycin and 250 ng/ml Amphotericin B) (Gibco BRL, UK), which immediately after, was added on a monolayer of approximately 10⁶ cells of the MARC-145 line in a 25 cm² culture dish.

After 24 hours, the medium was removed and replaced by DMEM supplemented with 2% equine serum and the same 2% antibiotic-antimycotic solution. Depending on cell growth, the culture was resuspended every 4-7 days with the use of a cell "scraper", it was passed through a syringe and 25 G needle and was added to a new culture of 5x10⁵ MARC-145 cells. Again, the medium was removed after 24 hours and replaced by DMEM supplemented with 2% equine serum.

The cultures were maintained in a humid stove at 37° C and 5% CO₂ atmosphere and it was observed daily in a phase contrast microscope to view the parasite.

After 24 days PI and after four consecutive rounds of the culture, tachyzoites were observed in the cell culture that was then kept for successive rounds according to the described procedure until obtaining counts in Neubauer chamber equal to or greater than 10⁶ tachyzoites.

From this point, the culture was maintained as described by Perez-Zaballos et al., 2005, J. Parasitol., 91 (3):507-10.

### Example 2.- Genetic characterization of the Nc-Spain 1H isolate. Identification of microsatellite sequences

This example describes the identification and genetic characterization of the Nc-Spain 1H isolate, together with other isolates obtained from various hosts and for different geographic origins, enabling its identification and discrimination from the other isolates. For this purpose, 13 microsatellite sequences identified in the genome of *N. caninum* were amplified and sequenced from 10 different isolates, including the one object of the present invention.

For the identification of microsatellite sequences in the *N. caninum* genome, approximately 25,330 ESTs (*Expression Sequences Tags*) deposited in public databanks derived from the Nc-1 and Nc-Liv isolates of *N. caninum* were analysed with the computer programs *Tandem Repeat Occurrence Locator* (TROLL) (Castelo et al., 2002. Bioinformatics. 18:634-636) and *Tandem Repeats Finder* (TRF) (Benson 1999, Nucleic Acids Res. 27:573-580) which allowed the identification of 181 micro- and minisatellite sequences. To avoid the analysis of redundant sequences and identify possible homologous sequences in other organisms, the 181 sequences were analysed using the computer program BLASTN confirming the presences of 40 redundant sequences. Thus, 126 sequences were found which contained microsatellites resulting from the tandem repetition of the motive (TA)ₙ, 2 of the (AGA)ₙ, and 1 for the motives (TC)ₙ, (GT)ₙ, (GA)ₙ, (TTC)ₙ, (CCT)ₙ, (AAG)ₙ, (TACA)ₙ and (ATCT)n.

From the 126 original sequences, 13 microsatellite markers were selected following different criteria which included the possibility of designing primers for their subsequent amplification by PCR and sequencing, the number of tandem repetitions they presented, selecting those of greatest number, and their localization, outside the coding regions of the genome, with the purpose of selecting the most polymorphic ones. Table I describes the microsatellites used in this study as molecular markers. For each microsatellite selected, which were called as specified in Table I, specific primers were designed (Genotek, Bonsay Techonologies, Spain) for the amplification of PCR products of approximately 300 pb, which included the repetition motive and part of the sequences flanking them. Subsequently, these primers were applied for the amplification and sequencing of the microsatellites selected from the genomic DNA of ten *N. caninum* isolates from different hosts and geographic origin, including the isolate object of the invention, as shown in Table II.

The PCR reactions were performed in volumes of 50 µl composed of 0.2 µM of each primer, 200µM dNTPs (Sigma), 1 x buffer (75mM Tris pH 9, 20mM (NH4)2SO4, 50mM KCI,) (Biotools, Spain), 2mM MgCl2 (Biotools), 2 units of DNA polymerase (Biotools) and 50 ng of DNA mould. The reaction conditions were the following: 1 cycle at 94° C for 5 min, 35 cycles of 94° C/1 min, 60° C / 1 min, and 72º C / 1 min, with a final cycle of 72° C for 10 min. In each series of amplifications DNA obtained from the cells where the parasite was habitually maintained and H2O were included as negative control. The PCR products obtained were separated by electrophoresis in 2.5% agarose-ethidium bromide gels (NuSieve 3:1, Cambrex BioScience, USA) and viewed under UV light. Then, the products were purified with the Exo-SAP IT kit (USB, USA), following the manufacturer's instructions, and directly sequenced in both directions. The sequences were analysed using the computer program BioEdit Sequence Alignment Editor v.7.0.1 (Copyright© 1997-2004 Tom Hall, Ibis Therapeutics, Carlsbad CA 92008, USA) and deposited in the GenBank database with access number AY935165-AY935200 and AY937107-AY937178, except for the Nc-Spain 1H isolate, which is set down in Figure 1.

**TABLE I: Description of the microsatellites used in this study as markers.**

| Marker | Access no. | Source^{a} | Repetition motive^{b} | PCR (5'-3') Primers | | No of alleles |
|---|---|---|---|---|---|---|
| | | | | Direct | Inverse | |
| MS1A | CF260074 | Nc-Liv | (TA)₆₀ | ACGACGCCTTTTCAACTGTC | CGTCCGTTCTCCTCTCTCAG | 3 |
| | CF941843 | Nc-1 | (AT)₁₂ | | | |
| MS1B | CF260074 | Nc-Liv | | GGAGAACGGACGACGTTAAG | CCCACAAACTCCCTTTCCTC | 3 |
| | CF941871 | Nc-1 | (AT)₂₃ | | | 4 |
| MS2 | CF261297 | Nc-Liv | | CTCTTCTCACCAGCCCAGTC | GCTGTTACAACCCACGGAAC | |
| | CD667923 | Nc-1 | (AT)₁₄ | | | 4 |
| MS3 | CF423151 | Nc-Liv | (AT)₁₈ | GACTTTGGTTTGCCACTGTCG | TCCGACATCTACGGACATCG | 6 |
| | CF967777 | Nc-1 | (TA)₁₁ | | | 7 |
| MS4 | CF937799 | Nc-Liv | | AGAAGAAGAAACGCGGAATG | TCTGAAACGAATTCCCTTGG | |
| | CF942627 | Nc-1 | (TA)₁₂ | | | 5 |
| MS5 | CF598343 | Nc-Liv | | AATCAGGACTCCGTCACACC | CCAGAGAGTCCCACATCTC | 4 |
| | CD667615 | Nc-1 | (AT)₁₂ | | | |
| MS6A | CF942647 | Nc-1 | (TA)₁₅ | TCCGCGTGTGGTTTATCT | TGCGCATGCACGAATAATAG | 5 |
| MS6B | CF261131 | Nc-Liv | | GCACTCATGGGATTTTGTAGG | AAAAATCAATGCACCTGATCG | 6 |
| | CF942647 | Nc-1 | (AT)₁₇ | | | 9 |
| MS7 | CF371133 | Nc-Liv | (ACT)₇- | GATCCAACTCGGCGAGATAC | CGTTCCCTTCCCAAATCTTC | |
| | CD680118 | Nc-1 | | | | 3 |
| MS8 | CF938766 | Nc-Liv | (AGA)₁₂- | ACACTCGCCTTTCCTTTGTG | CACACAGGCCAGTTGAAAAG | 2 |
| | CF421954 | Nc-1 | (TGA)₉ | | | |
| MS10 | CF659321 | Nc-1 | | CTATCACAGCCGTGAGTGTTG | CGCGCTATCCTTTATTCT | |
| MS12 | CF937360 | Nc-Liv | (GT)₁₆ | CCAGCATTTCTTCTCCTT | CAAAAACGCACTTTCTTTCG | |
| MS21 | BF824453 | Nc-1 | (TACA)₁₀ | TGTGAGCATAACGGTGGTTC | TTTCTACCCTCCCCTTCACC | |

| | | | | | | |
|---|---|---|---|---|---|---|
| ^{a}ESTs from genebanks of complementary tachyzoites DNA ^{b}Repetition motive identified in the ESTs from the Nv-Liv isolate and, exceptionally, from the Nc-1 isolate, when it is only available | | | | | | |

**TABLE II: N. caninum isolates included in this study and the alleles obtained for the different microsatellites analysed-**

| Isolate | Host | Geographic origin | Reference | Alleles and analysis in multilocus of the different microsatellites studied | | | | | | | | | | | | |
|---|---|---|---|---|---|---|---|---|---|---|---|---|---|---|---|---|
| | | | | MS1A | MS1B | MS2 | MS3 | MS4 | MS5 | MS6A | MS6B | MS7 | MS8 | MS10 | MS12 | MS21 |
| Nc-Liv | Canine | UK | Barber et al. 1995, Parasitol, 111 (Pt5) 563-568 | 1 | 1 | 3 | 4 | 6 | 1 | 4 | 2 | 4 | 5 | 9 | 2 | 1 |
| Nc-SweB1 | Bovine | Sweden | Stenlund et al., 1997, Parasitol Res. 84:214-219 | 1 | 1 | 4 | 1 | 4 | 5 | 1 | 4 | 3 | 2 | 2 | 2 | 1 |
| Nc-Bahia | Canine | Brasil | Gondim et al., 2001, Vet. Parasitol. 101: 1-7 | 1 | 2 | 2 | 3 | 4 | 5 | 4 | 2 | 1 | 2 | 1 | 2 | 1 |
| Nc-PV1 | Bovine | Italy | Magnino et al., 1999, Vet.. Rec. 144 456 | 1 | 1 | 3 | 2 | 5 | 3 | 5 | 2 | 4 | 4 | 4 | 2 | 1 |
| Hammondia heydorni Berlin 1996 | Canine | Germany | Müller et al., 2001, Parasitol Res. 87: 883-885 | 2 | 1 | 2 | 2 | 2 | 2 | 2 | 1 | 1 | 6 | 3 | 3 | 1 |
| Nc-GER1 | Canine | Germany | Peters et al., 2000, Parasitol. Res. 86:1-7 | 1 | 3 | 3 | 2 | 3 | 4 | 3 | 2 | 3 | 2 | 8 | 3 | 1 |
| KBA1 | Bovine | Korea | Kim et al., 2000, Vet Parasitol. 90: 147-154 | 1 | 1 | 2 | 1 | 6 | 6 | 3 | 2 | 2 | 3 | 5 | 2 | 1 |
| KBA2 | Ovine | Korea | Kim et al., 2000, Vet. Parasitol. 90: 147-154 | 3 | 1 | 2 | 1 | 6 | 7 | 3 | 2 | 2 | 3 | 6 | 2 | 1 |
| Nc-SHEEP | Bovine | Japan | Koyama et al., 2001, J. Parasitol. 87:1486-1488 | 1 | 1 | 1 | 2 | 1 | 1 | 4 | 3 | 2 | 1 | 7 | 1 | 1 |
| Nc-SPAIN 1H | BOVINE | SPAIN | | 1 | 1 | 3 | 2 | 4 | 1 | 6 | 2 | 3 | 4 | 6 | 2 | 1 |

One of the microsatellites was monomorphic in the 10 isolates analysed (MS21). The presence of polymorphism for the 13 microsatellites selected varied from a low polymorphism with 3 alleles for each microsatellite (MS1A, MS1B, MS12 and MS21) up to 4 to 9 alleles for the remaining microsatellites. Furthermore, MS7 showed an allele with SNP (*Single Nucleotide Polymorphism*).

To analyse the genotypes of each isolate in "multilocus", each allele was designated with a number in accordance with the length of the repetitive motive and the differences found in its sequences. The analysis in "multilocus" considerably increased the discriminatory power of the microsatellite sequences and thus, unique genetic profiles were obtained for each isolate, indicating that all the isolates differed significantly from one another, as can be observed in Table II.

### Example 3.- Pathogenicity of the Nc-Spain 1H isolate in a murine model

This section describes the experiments performed to investigate the pathogenicity of the Nc-Spain 1H isolate in an experimental murine model.

For this purpose, female BALB/c mice, of 6 weeks of age were inoculated intraperitoneally with 10⁵ (group A), 10⁶ (group B) and 10⁷ (group C) tachyzoites of the Nc-Spain 1H isolate obtained from the cell culture prepared in Example 1. The control group (group D) consisted of BALB/c mice inoculated with 200 µl of PBS by intraperitoneal route.

In the course of the experiment three animals from each group were randomly sacrificed on days 1, 2, 4, 8, 16 and 32, with the exception of days 1, 2 and 4 in group C where four mice were sacrificed per day.

The animals were housed in plastic cages, in groups of 5 animals per cage, they were kept at an ambient temperature of 20-24° C subjected to a photoperiod of 12 hours of light and 12 hours of darkness. The fodder and the water were supplied *ad libitum.*

All the mice were examined daily to observe symptoms compatible with infection by *N. caninum* in mice (Lindsay & Dubey, 1989, J.Parasitol. 75:772-779; Lindsay & Dubey, 1990, J.Parasitol. 76:410-413; Lindsay et al., 1995, J.Parasitol. 81:313-315).

The mice were sacrificed in a CO₂ bell. Immediately after the sacrifice, blood was obtained by intracardiac punction with an insulin syringe and 0.5x16 mm diameter needle. The mice were weighed at the time of sacrifice in order to detect a significant reduction in weight with respect to the control group. The blood samples (200-500 µl) were deposited in 1.5 ml tubes with EDTA to study the parasitaemia. Next, the autopsy of each individual was performed, opening the abdominal and thoraxic cavities, evaluating the presence of macroscopic lesions and extracting the lung and the brain, which were frozen at -80° C in a sterile 1.5 ml tube until the DNA was extracted in order to detect the presence of the parasite using PCR.

The blood samples were centrifuged at 2000g for 10 min, collecting the supernatant (plasma) which was conserved at -80º C until the humoral immune response study induced by the parasite using IIF and ELISA techniques.

The sediment formed by the blood cells, obtained after centrifuging the blood samples and removing the plasma, was used for DNA extraction using the commercial system: "Genomic-Prep cell and blood DNA isolation kit" (Amershan Biosciences), which includes the necessary reagents and instructions for its use.

The DNA samples were stored at -20° C until later analysis by PCR.

DNA extraction from the lungs and brain was performed from 10-20 mg of fresh frozen tissue, broken up with a scalpel, using the commercial system: "Genomic-Prep cell and tissue DNA isolation kit" (Amershan Biosciences) as a method, which includes the necessary reagents and instructions for its use.

The nested PCR technique of the ITS-1 region was used to detect the parasite in the blood, lungs and brain samples collected from the sacrificed animals, following the procedure described in Example 1. The DNA obtained from *N*. *caninum* tachyzoites in all the reactions was used as positive control and ultrapure sterile water as negative control.

The mice of the infected groups (groups A, B and C) did not develop signs compatible with infection or mortality, for the duration of the experiment. Splenomegaly and lymphadenopathy were observed during the autopsy in mesenteric and iliac ganglia in most of the mice infected with the parasite from day 2 to day 16 Pl.

*N. caninum* DNA was detected in blood from 1 to 5 PI (post-inoculation) and in the lungs and brain from day 1 to 16 PI. With regard to the detection frequency of *N. caninum,* greater detectability of the parasite was observed on increasing the dose of inoculation. In the Group D (control group) all the samples analysed were negative, in accordance with that expected.

The results obtained for the distribution of the parasite in Groups A, B and C are shown in Table III.

**TABLE III**

| Days PI | Blood | | | Lungs | | | Brain | | |
|---|---|---|---|---|---|---|---|---|---|
| | 10^{5a} | 10⁶ | 10⁷ | 10⁵ | 10⁶ | 10⁷ | 10⁵ | 10⁶ | 10⁷ |
| 1 | 1/3^{b} | 3/3 | 4/4 | 1/3 | 0/3 | 2/4 | 0/3 | 0/3 | 2/4 |
| 2 | 1/3 | 1/3 | 4/4 | 1/3 | 3/3 | 3/4 | 1/3 | 2/4 | 1/4 |
| 4 | 1/3 | 1/3 | 4/4 | 0/3 | 2/3 | 3/4 | 0/3 | 0/3 | 2/4 |
| 8 | 0/3 | 0/3 | 0/3 | 1/3 | 1/3 | 3/3 | 1/3 | 0/3 | 1/3 |
| 16 | 0/3 | 0/3 | 0/3 | 0/3 | 1/3 | 1/3 | 0/3 | 1/3 | 0/3 |
| 32 | 0/3 | 0/3 | 0/3 | 0/3 | 0/3 | 0/3 | 0/3 | 0/3 | 0/3 |

| | | | | | | | | | |
|---|---|---|---|---|---|---|---|---|---|
| ^{a} Dose of tachyzoites used in the inoculations. ^{b} The fractions represent number of mice positive for PCR / number of mice analysed. | | | | | | | | | |

In said table it can be observed that 8 days post-inoculation the parasite has disappeared from the mice blood, even from those that were inoculated with the highest dose, and 16 days post-inoculation it has disappeared from the lungs and the brain in most of the mice inoculated, even from those that were inoculated with the highest dose. This indicates a high degree of attenuation of the Nc-Spain 1H strain, object of this patent.

### Example 4.- Pathogenicity of the Nc-Spain 1H isolate in a gestating bovine model

This example describes the experiments performed to investigate the pathogenicity of the Nc-Spain 1H isolate in a gestating bovine model.

In this experiment, 13 heifers were inoculated on day 70 of gestation endovenously administering 10⁷ tachyzoites of the Nc-1 (group 1), Nc-Spain 1H (group 2) or PBS (group 3) isolate. Each group was composed of five animals, with the exception of group 3 which was three. The 13 gestating heifers were under 24 months of age, seronegative to *N. caninum, Leptospira spp.* and *Brucella abortus* and vaccinated against VIBR, VBVD, VPI 3 and VBRS.

For the inoculations, the tachyzoites of the Nc-1 and Nc-Spain 1H isolates were obtained by replication in cell culture as described in Example 1.

To monitor the gestation, a transrectal ecography was performed once a week, after the inoculation, in order to determine the feasibility of the foetus, sacrificing those animals where foetal death was detected by ecography. All the remaining heifers were sacrificed on day 45 post-inoculation (PI).

Whole blood was collected *in vivo* from the coccygeal vein in tubes with EDTA to study parasitaemia. The extraction of DNA, from blood and tissues of heifers and foetuses was performed using a commercial test (REALPURE, Durviz). Once the DNA was extracted, the concentration of each sample was measured by spectrophotometry adjusting the concentration to 60 ng/µl in sterile ultrapure water.

The histological study of the samples taken post-mortem was based on the observation of the lesions characteristic of infection by *N. caninum* in the bovine species. Lesions in the brain, heart, liver, and placenta were graduated to the extension and intensity of the lesion that occurred in absent (0), mild (1), moderate (2) or severe (3).

The clinical signs observed during the experiment were diarrhoea, fever and foetal death. The diarrhoea was observed on days 6 (animal 1776) and 7 PI (animals 8862 and 1776), probably due to the arrival of a new lot of fodder.

An abnormal increase in the temperature was not observed on any days when comparing the temperature between the different groups (P>0.05; Analysis of unifactorial variance).

Foetal death was diagnosed by ecography in 3 of the 5 animals belonging to group 1. The animals and days on which foetal death was diagnosed were the following: 7848 on day 26 PI, 1785 and 1767 on day 34 PI. One day 35 PI, the presence of amber coloured mucus was observed in the vulva and cervix of animal 1785. The remaining animals from group 1 and all animals from groups 2 and 3 showed a normal ecographic image with foetal movement.

With regard to parasitaemia, in all the animals of group 1 the presence of the parasite's DNA was observed in some point of the study, whilst in group 2, three of the five animals which formed it showed parasitaemia. In group 3, no animal was positive during the study following that expected.

Table IV shows the detection of the presence of *N. caninum* DNA in the blood of the animals infected with the isolate Nc-1 (group 1) and Nc-Spain 1H (group 2).

**Table IV**

| Day PI | Group 1 (Nc-1) | | | | | Group 2 (Nc-Spain1H) | | | | |
|---|---|---|---|---|---|---|---|---|---|---|
| | 1767 | 1785 | 1587 | 1786 | 7848 | 1711 | 1638 | 1563 | 1782 | 1965 |
| 3 | - | - | - | - | - | - | - | - | - | + |
| 5 | - | - | + | + | - | - | - | - | - | + |
| 10 | - | - | - | - | - | - | - | - | - | - |
| 12 | - | - | - | - | + | - | - | - | - | - |
| 17 | - | - | - | - | - | - | - | - | - | - |
| 19 | - | + | - | - | - | - | - | - | + | - |
| 24 | + | - | - | - | - | - | - | - | - | - |
| 26 | - | - | - | - | - | - | - | - | - | - |
| 31 | - | - | - | - | - | - | - | - | - | - |
| 34 | - | - | - | - | - | - | - | - | - | - |
| 39 | - | - | - | - | - | - | - | - | - | - |
| 41 | - | - | - | - | - | + | - | - | - | - |

During the external examination and autopsy of those animals wherein foetal death was diagnosed (7848, 1767 and 1785) a generalized ganglion hypertrophy was observed, in addition to reactive tonsils with marked activation of the white splenic pulp and hyperplasia of lymphoid follicles. In animal 7848, the lungs showed a variable quantity of foam in the trachea (pulmonary edema) and an edema was observed in the mucous in the abomasums and small intestine, more evident in the folds of the antral zone in the case of the abomasum, accompanied by an increase in mucus in the lumen (catarrhal abomasitis). In the remaining heifers, the lungs did not collapse as the negative pressure disappeared.

When the placenta was opened in the three animals sacrificed, a cloudy content of haemorrhagic appearance was observed. The placentomas were separated it being possible to observe the haemorrhagic caruncles, being more intense in animal 7848. The other heifers sacrificed on day 45 PI showed no noteworthy macroscopic alterations, with amniotic liquid and placentomas of normal appearance.

With regard to the results of the histological examination in the central nervous system of the heifers, the majority thereof did not show signs of inflammation with the exception of marked vascular congestion. Only in the animals with foetal death (animals 7848, 1767 and 1785) was the presence of some perivascular cuffs observed. In the placenta, two groups can histologically be identified. On the one hand, the lesions observed in the group of animals with foetal death wherein the placentoma showed an extensive haemorrhage in the upper part of the caruncle, in addition to multiple foci of necrosis in the maternal placenta. The majority of the foetal villus appeared necrotic with strong desquamation of the epithelium that covers it.

In relation to the intensity of the lesions in the heifers, the most severe were observed in the placenta and in the animals that showed foetal death.

Table V shows the intensity of the lesions observed in the heifers in the central nervous system (CNS) and in the placenta.

In relation to the macroscopic examination of the foetuses, macroscopic alterations were only observed in the three foetuses that died before day 45 PI. The foetuses showed a slightly autolytic appearance characterized by a generalized edema, as well as marked haemoperitoneum, haemothorax and haemopericardium. The remaining foetuses of the heifers sacrificed on day 45 PI did not show noteworthy macroscopic alterations.

**TABLE V**

| Animal reference | CNS | Placenta |
|---|---|---|
| 8862 | 0 | 0 |
| 1776 | 0 | 0 |
| 1560 | 0 | 0 |
| 1767 | 1 | 3 |
| 1785 | 0 | 3 |
| 1587 | 1 | 0 |
| 1786 | 0 | 2 |
| 7848 | 1 | 3 |
| 1711 | 0 | 0 |
| 1638 | 0 | 1 |
| 1563 | 0 | 0 |
| 1782 | 0 | 0 |
| 1965 | 0 | 0 |

In the histological study of the foetal organs, intense haematopoeietic activity was observed in the liver of all the foetuses, and in some of them focal accumulations of round intraparenchymatous cells (lymphocytes and histiocytes) which form small granuloma and marked sinus congestion. In the foetuses corresponding to animals 7848, 1767 and 1785 multiple foci of intraparenchymatous necrosis was observed (infectious necrotic hepatitis).

In the heart, the miscarried foetuses showed lymphocytic myocarditis, whilst in the other animals a slight diffusion of round cells and the presence of perivascular cuffs were observed. In the CNS, the most noteworthy lesions were the presence of microhaemorrhages, foci of perivascular lymphocytic cuffs and glial nodules.

In relation to the intensity of the lesions, the most severe were observed in the CNS and in the animals with foetal death. Likewise, a second evaluation was performed of the histologic lesions in the infected groups.

Table VI shows the intensity of the lesions observed in the foetuses in the liver, heart and the central nervous system.

**TABLE VI**

| Animal reference | Liver | Heart | CNS |
|---|---|---|---|
| 8862 | 0 | 0 | 0 |
| 1776 | 0 | 0 | 0 |
| 1560 | 0 | 0 | 0 |
| 1767 | 2/3^{a} | 2/2 | 1/1 |
| 1785 | 1/1 | 2/1 | 2/2 |
| 1587 | 1/1 | 1/1 | 2/1 |
| 1786 | 1/0 | 1/1 | 3/2 |
| 7848 | 3/2 | 2/2 | 2/2 |
| 1711 | 0/0 | 1/1 | 1/1 |
| 1638 | 0/0 | 1/0 | 2/1 |
| 1563 | 0/0 | 1/1 | 1/1 |
| 1782 | 0/1 | 2/1 | 1/1 |
| 1965 | 0/0 | 1/0 | 2/0 |

| | | | |
|---|---|---|---|
| ^{a}Results of the second evaluation of the histological lesions | | | |

The presence of the parasite DNA was not detected in any of the brain samples of the different heifers analysed, nevertheless, when the placentas were studied, those from the miscarried animals were positive and also those of heifer 1638 (group Nc-Spain1H). Likewise, the presence of the parasite was detected in the brain, heart and liver of the miscarried foetuses (foetuses 7848, 1767 and 1767). The results were negative in the remaining foetuses.

### Example 5.- Immunogenicity of the Nc-Spain 1H isolate in a murine model

The detection of specific antibodies against *N. caninum* in the plasma of the infected mice of Example 3 was determined using IIF and ELISA techniques.

For the IIF technique (Trees et al., 1993. Vet.Rec. 132:125-126), initially 8 µl of a suspension of 10⁷ formalinized tachyzoites/ml of the reference isolate Nc-1 (Dubey et al., 1988, J. Am. Vet. Med. Assoc. 193:1259-63) were deposited in each well of an immunofluorescence slide (Cultek) of 18 wells of 4 mm diameter. Next, the preparation was left in the air, then being fixed with acetone during 10 min at - 20° C. After the fixation, the serum from the infected mice (10 µl/well) was added, and it was incubated for 30 min at 37° C in a humid chamber.

The dilutions of the mice serums were carried out in PBS starting with a 1:25 dilution and making serum dilutions until achieving the last dilution wherein the serums stopped being positive. As negative control of the reaction, serums were used from the mice with PBS and PBS was used as blank of the reaction. Next, the slide was washed twice with PBS (10 min each time) and the IgG mouse immunoglobulin was added bound to fluoroscein isothiocyanate fluorochrome (Sigma, reference F-0257) at a 1:128 dilution in a PBS-Evans blue solution (1:10000), being incubated at 37° C during half an hour in a humid chamber. Next, the wells were again washed with PBS (3 times during 10 min each time) performing the last wash for 10 min with distilled water, after which, the wells were dried in the air and the slide was mounted with Fluoprep (Biomerieux) and a 24 x 60 mm slide cover. The wells were examined with a fluorescence microscope at x 400 (Nikon, Mercury lamp model HB-10101AF).

The presence of antibodies was detected from day 8 PI in the groups inoculated with the highest dose (group C), as shown in Table VII.

**TABLE VII**

| Days PI | DOSE | | |
|---|---|---|---|
| | 10⁵ | 10⁶ | 10⁷ |
| 1 | 0/3^{a} | 0/3 | 0/4 |
| 2 | 0/3 | 0/3 | 0/4 |
| 4 | 0/3 | 0/3 | 0/4 |
| 8 | 0/3 | 0/3 | 3/3 |
| 16 | 0/3 | 3/3 | 3/3 |
| 32 | 1/3 | 1/3 | 3/3 |

| | | | |
|---|---|---|---|
| ^{a} The fractions represent number of positive mice by IIF/ number of mice analysed. | | | |

The titres obtained by IIF also varied in accordance with the infective dose, the highest one being obtained (1:200-1:400) in the mice of group C, as shown in Figure 2.

The detection of the immunoglobulins of the IgG1 and IgG2a isotypes specific for *N. caninum* in the plasma was performed by an indirect immunoenzymatic assay (ELISA) following conventional procedures. The results were expressed as optical density. As controls of the assay, a negative sample of mouse serum was included on each plate (optical density less than 0.1) as well as a mouse serum experimentally infected with an optical density over 2.0.

In relation to the IgG2a and IgG1 isotopes, an increase thereof was observed from day 8 PI, the increase in immunoglobulin IgG2a and in groups B and C being observed. On days 16 and 32 PI, the mice inoculated with 10⁵ and 10⁶ tachyzoites had a greater concentration of IgG2a than IgG1. Nevertheless, in the group inoculated with 10⁷ tachyzoites, the production of IgG1 was predominant on day 16 (Figures 3A, 3B and 3C).

### Example 6.- Immunogenicity of the Nc-Spain 1H isolate in a gestating bovine model

In this test, 13 heifers under 24 months of age were used that were seronegative to *N. caninum,* IBRv, BVDv, *Leptospira spp.* and *Brucella abortus.*

On day 70 of gestation, 10⁷ tachyzoites of the Nc-1 isolate (group 1) were administered intravenously to 5 animals, 10⁷ tachyzoites of the Nc-Spain 1H isolate (group 2) to another 5 animals, or PBS (group 3, control group) to another three animals.

Whole blood was collected from the coccygeal vein in heparinized tubes to study the production of IFN-γ and in tubes without anticoagulant to obtain serum that was used in the study of the humoral immune response (IgG, IgG1 and IgG2) to *N*. *caninum* by ELISA. The samples were collected on days -2 and 0 and then 2 times a week from day 3 to day 41 post-inoculation (PI).

To determine the production of IFN-γ by sensitized peripheral lymphocytes, the blood samples were stored at ambient temperature and the culture process was started immediately after its obtainment. The entire process was performed in a laminar flow cabin using micropipette points with a filter.

The blood in the heparinized tubes was homogenized 10 times, and 900 µl of whole blood was added to each well of a flat-bottom culture plate (3 wells per animal). A well was stimulated with Concanavalina A (100 µl, final concentration 10µg/ml, Sigma), a second with soluble Nc-1 antigen (100 µl, final concentration 1µg/ml) and the third with sterile PBSI (100 µl, 0.01 M, pH 7.2). Next, the plates were homogenized 20 times and they were incubated in a stove at 37° C in an atmosphere with 5% CO₂. After 20-24 h, the plates were centrifuged at 500g, 10 min at ambient temperature and 200-400µl of the supernatant was collected from each well which were stored in 1.5 ml tubes at -20° C until their later analysis. The determination of IFN-γ was performed from the supernatants using a commercial ELISA assay (Bovigam, CSL, Ingenasa) following the manufacturer's indications. The results were expressed as values of optical density (OD) at 450 nm.

An increase in IFN-γ levels was detected in the infected groups compared to those of the control group (group 3) on day 5 PI (P<0.05; Duncan). In the group infected with Nc-1 (group 1) high levels of IFN-γ were observed throughout the study, reaching maximum levels on day 24 PI. In the group infected with Nc-Spain 1H (group 2), the highest values were observed on days 5 and 12 PI, the time after which a decrease was observed in IFN-γ levels, only finding significant differences with respect to the control group on days 19 and 34 PI (P<0.05; Duncan).

When the two infected groups were compared, the values obtained in group 1 were significantly superior to those of group 2 on days 10, 12, 17, 19, 24, 31, 34, 39 and 41 PI (P<0.05; Duncan) as can be observed in Figure 4.

The characterization of the humoral immune response (total IgG, IgG1 and IgG2a) was performed using an indirect ELISA assay with soluble *N. caninum* proteins as antigen.

The serum samples were diluted to 1:100. As positive control the serum was used of from reproducer with miscarriage associated to infection by *N. caninum* with positive serology results by WB and IIF (titre 1:1600-3200). As negative control, the serum from a reproducer was used from a farm without a history of reproductive failure and negative serology results by WB and IIF.

To determine total IgG, a monoclonal mAb anti bovine IgG1 and IgG2 antibody (Laboratoire Service International, France) was used at a dilution of 1:2000 in 0.05%PBS-Tween. The conjugates for the evaluation of IgG1 and IgG2 (Serotec) were used at a dilution of 1:100000 and 1:40000, respectively. The results were expressed as values of optical density at 450 nm (OD).

On analysing the results obtained for total specific IgG, in group 1 (Nc-1) a significant increase was observed in the OD values with respect to the control group on day 10 PI, but especially from day 17 PI (P<0.05; Duncan), obtaining the highest values on days 24 and 26 PI. In group 2 (Nc-Spain1H), an increase was also observed from day 17 PI with the maximum value on day 19 PI.

Nevertheless, despite the fact that the values from 2 were higher than those of the control group, significant differences were not obtained (P>0.05; Duncan). When the two infected groups are compared, the values obtained in group 1 were significantly higher than those of group 2 on days 17, 24, 26, 31, 34, 39 and 41 PI (P<0.05; Duncan).

Figure 5 shows the OD values for the three groups.

The isotypes IgG1 and IgG2 were evaluated from the day on which a significant increase in the OD values of IgG with respect to the control group were observed (day 10 PI). The IgG1 levels remained at the same level as the control group during the first days PI analysed. In both groups, a significant increase was detected versus the control group on day 12 PI (P<0.05; Duncan) and the highest levels were attained from day 34 PI. With respect to IgG2 values, in group 1 a significant increase was detected compared with the control group on day 17 PI (P<0.05; Duncan), reaching the maximum levels on days 34 and 39 PI. In group 2, the IgG2 values remained slightly above the control group, not finding, however, significant differences between both (P>0.05; Duncan).

In the two infected groups, the IgG1 concentration was greater than the IgG2 concentration during the experiment. When the two infected groups were compared, the values of both isotypes were higher in group 1 than in group 2 from day 17 PI (P<0.05, Duncan). The control animals remained seronegative to *N. caninum* throughout the experiment.

Figures 6A and 6B represent the mean values of IgG1 and IgG2 anti-*N*. *caninum* in the animals inoculated with the isolate Nc-1, Nc-Spain 1 H, and PBS.

In said figures, the production of antibodies against *N. caninum* proteins was detected in the animals infected with the isolate of the invention.

## Claims

1. Parasite isolate deposited in the Culture Collection of Algae and Protozoa (CCAP) with access number CCAP 2051/2.

2. Biologically pure cell culture infected by the isolate of claim 1.

3. Cell culture of claim 2, **characterized in that** it is from monkey kidney cells.

4. Antibody which reacts specifically against an antigen from the isolate of claim 1, **characterized in that** the antigen is selected from the group formed by the isolate, tachyzoites, bradyzoites, and sporozoites.

5. Antibody according to claim 4, **characterized in that** the antigen is a tachyzoite.

6. Antibody according to any of claims 4 to 5, **characterized in that** it is monoclonal.

7. Vaccine which comprises the isolate of claim 1.

8. Vaccine according to claim 7, **characterized in that** the isolate is in the form of live attenuated isolate, inactivated or fixed, at any stage of its biological cycle.

9. Use of the isolate of claim 1 to prepare a vaccine for the treatment and/or prevention of neosporosis in animals.

10. Use of antibodies according to any of claims 4 to 6 to prepare a drug for the treatment of an infection and/or disease caused by *Neospora caninum* in animals.

11. Use of antigens from the isolate of claim 1 to detect infection by the parasite *Neospora caninum* in animals, from a biological sample, **characterized in that** the antigen is selected from the group formed by the isolate, tachyzoites, bradyzoites, and sporozoites.

12. Use according to claim 11, **characterized in that** the antigen is a tachyzoite.

13. Use of antibodies according to any of claims 4 to 6 to detect infection by the parasite *Neospora caninum* in animals, from a biological sample.

## Patentansprüche

1. Parasitenisolat, welches bei der Culture Collection of Algae and Protozoa (CCAP, Kultursammlung von Algen und Protozoen) mit der Hinterlegungsnummer CCAP 2051/2 hinterlegt ist.

2. Biologisch reine Zellkultur, welche mit dem Isolat nach Anspruch 1 infiziert ist.

3. Zellkultur nach Anspruch 2, **dadurch gekennzeichnet, dass** sie von Affen-Nierenzellen ist.

4. Antikörper, welcher spezifisch gegen ein Antigen aus dem Isolat nach Anspruch 1 reagiert, **dadurch gekennzeichnet, dass** das Antigen aus der Gruppe gewählt wird, welche aus Folgendes besteht: das Isolat, Tachyzoiten, Bradyzoiten und Sporozoiten.

5. Antikörper nach Anspruch 4, **dadurch gekennzeichnet, dass** das Antigen ein Tachyzoit ist.

6. Antikörper nach einem der Ansprüche 4 bis 5, **dadurch gekennzeichnet, dass** er monoklonal ist.

7. Impfstoff, welcher das Isolat nach Anspruch 1 umfasst.

8. Impfstoff nach Anspruch 7, **dadurch gekennzeichnet, dass** das Isolat in Form eines abgeschwächten, inaktivierten oder fixierten, Lebendisolats, in irgendeine Phase seines biologischen Zyklus ist.

9. Verwendung des Isolats nach Anspruch 1, um einen Impfstoff für die Behandlung und/oder die Vorbeugung von Neosporose in Tieren herzustellen.

10. Verwendung von Antikörpern nach einem der Ansprüche 4 bis 6, um ein Arzneimittel für die Behandlung einer von *Neospora caninum* in Tieren verursachten Infektion und/oder Krankheit herzustellen.

11. Verwendung von Antigenen aus dem Isolat nach Anspruch 1, um eine Infektion durch den Parasiten *Neospora caninum* in Tieren, aus einer biologischen Probe nachzuweisen, **dadurch gekennzeichnet, dass** das Antigen aus der Gruppe gewählt wird, welche aus Folgendes besteht: das Isolat, Tachyzoiten, Bradyzoiten und Sporozoiten.

12. Verwendung nach Anspruch 11, **dadurch gekennzeichnet, dass** das Antigen ein Tachyzoit ist.

13. Verwendung von Antikörpern nach einem der Ansprüche 4 bis 6, um eine Infektion durch den Parasiten *Neospora caninum* in Tieren, aus einer biologischen Probe nachzuweisen.

## Revendications

1. Isolat parasitaire déposé au CCAP (Culture Collection of Algae and Protozoa) sous le numéro d'accès CCAP 2051/2.

2. Culture cellulaire biologiquement pure infectée par l'isolat de la revendication 1.

3. Culture cellulaire selon la revendication 2, **caractérisée en ce qu'**elle provient de cellules de rein de singe.

4. Anticorps qui réagit spécifiquement contre un antigène de l'isolat de la revendication 1, **caractérisé en ce que** l'antigène est sélectionné parmi le groupe constitué par l'isolat, des tachyzoïtes, des bradizoïtes et des sporozoïtes.

5. Anticorps selon la revendication 4, **caractérisé en ce que** l'antigène est un tachyzoïte.

6. Anticorps selon l'une quelconques des revendications 4 à 5, **caractérisé en ce qu'**il est monoclonal.

7. Vaccin qui comprend l'isolat de la revendication 1.

8. Vaccin selon la revendication 7, **caractérisé en ce que** l'isolat est sous forme d'isolat vivant atténué, inactivé ou fixé, à n'importe quelle phase de son cycle biologique.

9. Utilisation de l'isolat de la revendication 1 pour préparer un vaccin pour le traitement et/ou la prévention de la néosporose chez les animaux.

10. Utilisation d'anticorps selon l'une quelconque des revendications 4 à 6 pour préparer un médicament pour le traitement d'une infection et/ou maladie causée par *Neospora caninum* chez les animaux.

11. Utilisation d'antigènes à partir de l'isolat de la revendication 1 pour détecter l'infection par le parasite *Neospora caninum* chez les animaux, a partir d'un échantillon biologique, **caractérisée en ce que** l'antigène est sélectionné parmi le groupe formé par l'isolat, les tachyzoïtes, les bradizoïtes et les sporozoïtes.

12. Utilisation selon la revendication 11, **caractérisée en ce que** l'antigène est un tachyzoïte.

13. Utilisation d'anticorps selon l'une quelconque des revendications 4 à 6 pour détecter l'infection par le parasite *Neospora caninum* chez les animaux, à partir d'un échantillon biologique.
